(19) Europäisches Patentamt
European Patent Office
Office européen des brevets

(11) EP 0 735 500 A2

(12) **EUROPÄISCHE PATENTANMELDUNG**

(43) Veröffentlichungstag:
02.10.1996 Patentblatt 1996/40

(51) Int. Cl.⁶: **G06F 19/00**

(21) Anmeldenummer: 96103984.9

(22) Anmeldetag: 13.03.1996

(84) Benannte Vertragsstaaten:
**DE FR GB**

(30) Priorität: **29.03.1995 DE 19511532**

(71) Anmelder: **SIEMENS AKTIENGESELLSCHAFT**
**80333 München (DE)**

(72) Erfinder:
• **Bruder, Herbert, Dr.**
**91315 Höchstadt (DE)**
• **Killmann, Reinmar, Dr.**
**91301 Forchheim (DE)**

(54) **Verfahren zum Lokalisieren einer elektrischen Herzaktivität**

(57) Bei einem Verfahren zum Lokalisieren einer elektrischen Herzaktivität werden mit einem Vielkanal-meßsystem (4,6) von der Herzaktivität erzeugte Körperoberflächenpotentiale an mehreren Meßpunkten gemessen und Werte gespeichert, die die Körperoberflächenpotentiale an den Meßpunkten charakterisieren. Die Werte werden dann mit in einer Datenbank (8) abgespeicherten Vergleichs-Werten verglichen, wobei die Vergleichs-Werte Vergleichs-Oberflächenpotentiale charakterisieren, die von Vergleichs-Herzaktivitäten herrühren, deren Lage im Herzen bekannt ist. Die Lage derjenigen Vergleichs-Herzaktivität wird als Lokalisierungsergebnis der Herzaktivität ausgegeben, deren Vergleichs-Werte mit den charakteristischen Werten die größte Ähnlichkeit aufweisen. Erfindungsgemäß sind die Vergleichswerte mit Hilfe eines in einem Thoraxmodell (14) angeordneten Herzmodells (12) ermittelt worden.

FIG 1

**Beschreibung**

Die Erfindung betrifft ein Verfahren zum Lokalisieren einer elektrischen Herzaktivität, bei dem

-   mit einem Vielkanalmeßsystem von der Herzaktivität erzeugte Körperoberflächenpotentiale an mehreren Meßpunkten gemessen werden und Werte gespeichert werden, die die Körperoberflächenpotentiale an den Meßpunkten charakterisieren,
-   die Werte mit in einer Datenbank abgespeicherten Vergleichs-Werten verglichen werden, wobei die Vergleichs-Werte Vergleichs-Oberflächenpotentiale charakterisieren, die von Vergleichs-Herzaktivitäten herrühren, deren Lage im Herzen bekannt ist, und
-   die Lage derjenigen Vergleichs-Herzaktivität als Lokalisierungsergebnis der Herzaktivität ausgegeben wird, deren Vergleichs-Werte mit den charakteristischen Werten die größte Ähnlichkeit aufweisen.

Ein Verfahren der eingangs genannten Art ist aus dem Artikel von SippensGroenewegen et al. mit dem Titel: "Localization of the Site of Origin of Postinfarction Ventricular Tachycardia by Endocardial Pace Mapping", erschienen in Circulation, Vol. 88, No. 5, Part 1, November 1993, pp. 2290-2306, bekannt. Körperoberflächenpotentiale von elektrischen Herzaktivitäten werden an der Thoraxoberfläche mit 62 Elektroden gleichzeitig abgegriffen. Die Signale werden über den QRS-Komplex integriert. Als charakteristischer Wert wird dann der integrierte Meßwert mit entsprechenden Vergleichs-Signalen, die von Vergleichs-Herzaktivitäten mit bekannter Lage herrühren, verglichen. Als Lokalisierungsergebnis wird die Lage derjenigen Vergleichs-Herzaktivität ausgegeben, deren Vergleichs-Werte am besten mit den charakteristischen Werten übereinstimmen.

In dem Artikel von SippensGroenewegen et al. mit dem Titel: "Body Surface Mapping of Ectopic Left and Right Ventricular Activation", erschienen in Circulation, Vol. 82, No. 3, September 1990, pp. 879-896, ist genauer angegeben, wie die Vergleichs-Werte von Vergleichs-Herzaktivitäten erzeugt werden. Danach werden an gesunden Personen mit einer Vielfach-Elektroden-Anordnung Vergleichs-Oberflächenpotentiale gemessen, die von einem Stimulations-Katheter im Herzen erzeugt werden. Der Ort des Katheters wird bestimmt über eine biplanare cineradiographische Methode. Die Zusammenstellung einer ausreichend großen Anzahl von Vergleichs-Herzaktivitäten und den dazugehörigen Vergleichs-Körperoberflächenpotentialen zur Lokalisierung ist wegen den erforderlichen Messungen an gesunden und kranken Patienten sehr aufwendig.

Aus dem Artikel von Killmann et al. mit dem Titel: "Three-dimensional computer model of the entire human heart for simulation of reentry and tachycardia: gap phenomenon and Wolff-Parkinson-White syndrome", erschienen in Basic Research in Cardiology, Vol. 86, 1991, pp. 485-501, ist ein Computermodell zur Simulation von normalen und pathologischen EKG-Daten bekannt. In dem Herzmodell ist das Herz in Volumenzellen aufgeteilt, die entsprechend der Herzphysiologie zusammenhängen. Jeder Zelle sind elektrophysiologische Parameter zugeordnet. Insbesondere werden die Parameter Erregungsausbreitungsgeschwindigkeit, Refraktärzeit und Aktionspotentialform den Zellen zugeordnet. Sie können im Rahmen des physiologisch Sinnvollen frei gewählt werden. Ausgehend von einer Erregung am Sinusknoten werden darauffolgend bei der Erregungsausbreitung die einzelnen Volumenzellen aktiviert entsprechend den ihnen zugeordneten elektrophysiologischen Parametern. Die Erregungsausbreitung ist begleitet von einem elektrischen Feld, aus dem Körperoberflächenpotentiale berechnet werden können. Die Zellen haben eine Ausdehnung von ca. 2,5 mm.

Ein Verfahren zur Berechnung von Vergleichs-Oberflächenpotentiale ist in dem Artikel von Bömmel et al. "Boundary Element Solution of Biomagnetic Problems", IEEE Trans. Magn. MAG-29, 1993, pp. 1395-1398 beschrieben. Mit Hilfe eines modifizierten Boundary-Element-Verfahrens werden von einem Herzmodell erzeugte elektrische Werte zur Berechnung der Verteilung von Körperoberflächenpotentialen auf einem Thoraxmodell herangezogen.

Das Verfahren der Hauptkomponentenzerlegung, wie es z. B. in dem Artikel Wold et al. "Principal Component Analysis", erschienen in Chemometrics and Intelligent Laboratory Systems, Vol. 2, 1987, pp. 37-52, beschrieben ist, kann benutzt werden, große Datenmengen auf ihren charakteristischen Informationsinhalt zu reduzieren.

DE 43 07 545 A1 offenbart ein Gerät und ein Verfahren zur Bestimmung des Ortes und/oder der Ausdehnung von Ischämien und/oder Infarkten im Herzen eines Lebewesens. Dort werden EKG-Meßwerte einem neuronalen Netz zugeführt. Das neuronale Netz ist mit Hilfe des vorstehend schon erwähnten Herzmodells entsprechend trainiert, aus den EKG-Daten den Ort und/oder die Ausdehnung von krankhaften Veränderungen auszugeben. Der Vorbereitungsaufwand zur Realisierung eines neuronalen Netzes, das für die Lösung der Lokalisierungsaufgabe geeignet ist, ist jedoch hoch.

Der Erfindung liegt nun die Aufgabe zugrunde, ein Verfahren zum Lokalisieren einer elektrischen Herz-Aktivität anzugeben, mit dem eine ausreichend genaue und schnelle Lokalisierung durchgeführt werden kann.

Die Aufgabe wird dadurch gelöst, daß die Vergleichs-Werte mit Hilfe eines in einem Thoraxmodell angeordneten Herzmodells ermittelt worden sind. Damit können, ohne eine große Anzahl von Patienten untersuchen zu müssen, in einem ausreichend engen räumlichen Raster Vergleichs-Herzaktivitäten angeordnet und die dazugehörigen Vergleichs-Werte ermittelt werden. Die Vergleichs-Werte können dann in einer datenbankähnlichen Struktur abgespeichert werden.

Eine vorteilhafte Ausgestaltung zeichnet sich dadurch aus, daß zum Vergleich normierte Korrelationskoeffizienten aus den charakteristischen Werten mit den Vergleichs-Werten jeder Vergleichs-Herzaktivität gebildet werden, wobei die Lage der Vergleichs-Herzaktivität, deren Vergleichs-Werte mit den charakterisierenden Werten den größten Korrelationskoeffizienten bildet, als Lokalisierungsergebnis ausgegeben wird. Der normierte Korrelationskoeffizient der zu vergleichenden Funktionen ist ein für die Lösung der Lokalisierungsaufgabe ausreichendes Ähnlichkeitsmaß. Insbesondere haben Amplitudenunterschiede bei sonst gleichem Signalverlauf keinen Einfluß auf das Ähnlichkeitsmaß.

Bei einer weiteren vorteilhaften Ausgestaltung ist der Vergleich auf den QRS-Komplex in den Körperoberflächenpotentialen beschränkt. Pathologische elektrische Herzaktivitäten beeinflußen hauptsächlich den Verlauf der Körperoberflächenpotentiale im QRS-Komplex.

Bei einer weiteren vorteilhaften Ausgestaltung geben die charakteristischen Werte den Zeitverlauf der Körperoberflächenpotentiale an den Meßpunkten wieder. Dabei muß zwar eine große Anzahl von charakteristischen Werten mit einer entsprechenden Anzahl von Vergleichs-Werten miteinander korreliert werden, es wurde jedoch festgestellt, daß der zeitliche Verlauf der Vergleichs-Oberflächenpotentiale weniger empfindlich auf Variationen der Geometrie des Thoraxmodells reagiert. Abweichungen der Patientenanatomie vom gewählten Thoraxmodell haben daher einen nur geringen Einfluß auf die Genauigkeit des Lokalisierungsergebnisses.

Eine besonders vorteilhafte Ausgestaltung zeichnet sich dadurch aus, daß aus diskreten Werten des Zeitverlaufs der Körperoberflächenpotentiale an den Meßpunkten Komponenten eines Meßwertvektors gebildet werden, daß für jede Lage der Vergleichs-Herzaktivität ein entsprechender Vergleichsvektor vorliegt, daß der Meßwertvektor mit jedem Vergleichsvektor verglichen wird, indem jeweils ein auf die Beträge der beiden Vektoren normiertes Skalarprodukt der beiden Vektoren gebildet wird, und daß die Lage der Vergleichs-Herzaktivität, deren Vergleichsvektor mit dem Meßwertvektor das größte nomierte Skalarprodukt bildet, als Lokalisierungsergebnis ausgegeben wird.

Eine Datenreduktion kann nach einer weiteren besonders vorteilhaften Ausgestaltung dadurch erreicht werden, daß aus dem diskretisierten zeitlichen Verlauf von Vergleichs-Oberflächenpotentialen, die von Vergleichs-Herzaktivitäten herrühren, deren Lage im Herzen bekannt sind, Eigenvektoren extrahiert werden, daß die charakteristischen Werte Meß-Entwicklungskoeffizienten wiedergeben, die das Ergebnis einer spektralen Zerlegung des diskretisierten zeitlichen Verlaufs der an den Meßpunkten gemessenen Körperoberflächenpotentiale nach den Eigenvektoren darstellen, und daß die Vergleichs-Werte Vergleichs-Entwicklungskoeffizienten wiedergeben, die das Ergebnis einer spektralen Zerlegung des diskretisierten zeitlichen Verlaufs der Vergleichs-Oberflächenpotentiale sind.

Die Erfindung wird im folgenden anhand von zwei Figuren erläutert. Es zeigen:

FIG 1    eine erste Variante des Lokalisierungsverfahrens, wobei eine räumlich-zeitliche Korrelation durchgeführt wird und

FIG 2    eine zweite Variante des Lokalisierungsverfahrens, wobei eine Korrelation von Entwicklungskoeffizienten durchgeführt wird, die das Ergebnis einer spektralen Zerlegung nach Eigenvektoren darstellen.

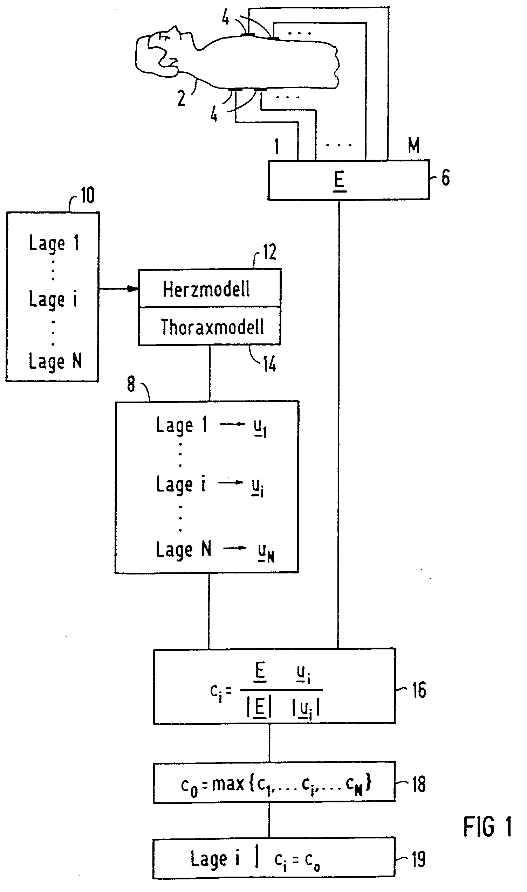

In der schematischen Darstellung der ersten Variante des Lokalisierungsverfahrens ist in FIG 1 ein Oberkörper eines Patienten 2 dargestellt, auf dem an der Vorder- und Rückseite mit Elektroden 4 an z. B. M = 64 Positionen die von Herzaktivitäten erzeugten Körperoberflächenpotentiale gemessen werden. Es wird hier der QRS-Komplex aus den Meßsignalverläufen ausgewertet, da sich viele Pathologien in abnormalen QRS-Signalen äußern. Die Meßwerte jeder Elektrode 4 oder jedes Meßkanals werden abgetastet, wobei hier bei einer Abtastfrequenz von 500 Hz typischerweise ca. K = 50-60 QRS Signalwerte erzeugt werden. Diese Werte werden als Komponenten eines Meßwertvektors $\underline{E}$ betrachtet. Der Meßwertvektor $\underline{E}$ besitzt demnach L = M x K = 64 x 50 Komponenten, die z. B. in der zeitlichen Reihenfolge, wie das Signal abgetastet wird, im Aquisitionsschritt 6 aufgefüllt oder gespeichert werden. Beispielsweise entspricht die erste Komponente des Meßwertvektors $\underline{E}$ dem Potentialwert am Meßkanal 1 zum ersten Abtastzeitpunkt, die zweite Komponente entspricht dem Meßwert des Potentials am Meßkanal 2 zum ersten Abtastzeitpunkt usw.

In einer Datenbank 8 sind Vergleichs-Werte $\underline{u}_i$ abgespeichert. Die Vergleichs-Werte $\underline{u}_i$ geben entsprechend den gemessenen Körperoberflächenpotentialen Vergleichs-Oberflächenpotentiale von Vergleichs-Herzaktivitäten wieder, deren Lage 10 im Herzen bekannt ist. Die Datenbank 8 enthält i = 1 bis i = N = 244 Vergleichs-Oberflächenpotentiale, was einem Rasterabstand im Herzmodell von ca. 0,75 cm bis 1 cm der Vergleichs-Herzaktivitäten entspricht. Die Vergleichs-Herzaktivitäten mit den dazugehörigen Vergleichs-Oberflächenpotentialen werden über ein Herzmodell 12 erzeugt, wie es in dem eingangs schon erwähnten Artikel von Killmann et al. beschrieben ist. Eingebettet ist das Herzmodell 12 in ein Thoraxmodell 14, das es erlaubt, die Vergleichs-Oberflächenpotentiale zu berechnen.

Ein geeignetes Thoraxmodell ist aus dem eingangs erwähnten Artikel von Bömmel et al. bekannt. Der Vorteil der Bestimmung der Vergleichs-Oberflächenpotentiale aus dem Herzmodell 12 im Thoraxmodell 14 liegt darin, daß in einer nahezu beliebigen Dichte die Orte der Vergleichs-Herzaktivitäten variiert werden und die dazugehörigen Vergleichs-Oberflächenpotentiale berechnet werden können. Die Dichte der Orte der Vergleichs-Herzaktivitäten kann insbesondere nach physiologischen Gesichtspunkten bestimmt werden. Bei der Berechnung der Vergleichs-Oberflächenpotentiale ist darauf zu achten, daß die Anordnung der Punkte an denen die Vergleichs-Oberflächenpotentiale berechnet werden mit der tatsächlichen Anordnung der Elektroden 4 der Vielkanalmeßanordnung übereinstimmen.

Über eine Korrelation 16 wird für jede Lage $i = 1$ bis $i = N$ der Vergleichs-Herzaktivität ein normierter Korrelationskoeffizient $c_i$ gebildet wird, der das Skalarprodukt der auf den Betrag normierten Vergleichs-Potentialwerte $\underline{u}_i/|\underline{u}_i|$ mit dem auf den Betrag normierten Meßwertvektor $\underline{E}/|\underline{E}|$ darstellt. Liegen alle normierten Korrelationskoeffizienten $c_i$ vor, wird der größte Korrelationskoeffizient $c_0$ in einem Suchschritt 18 herausgesucht. Die zum Korrelationskoeffizient $c_0$ gehörende Lage der Vergleichs-Herzaktivität gibt das Lokalisierungsergebnis für die dem Meßwertvektor $\underline{E}$ zugrundeliegende Herzaktivität wieder. Eine Ausgabeeinheit 19 gibt die Lage der gefundenen Vergleichs-Herzaktivität als Lokalisierungsergebnis aus.

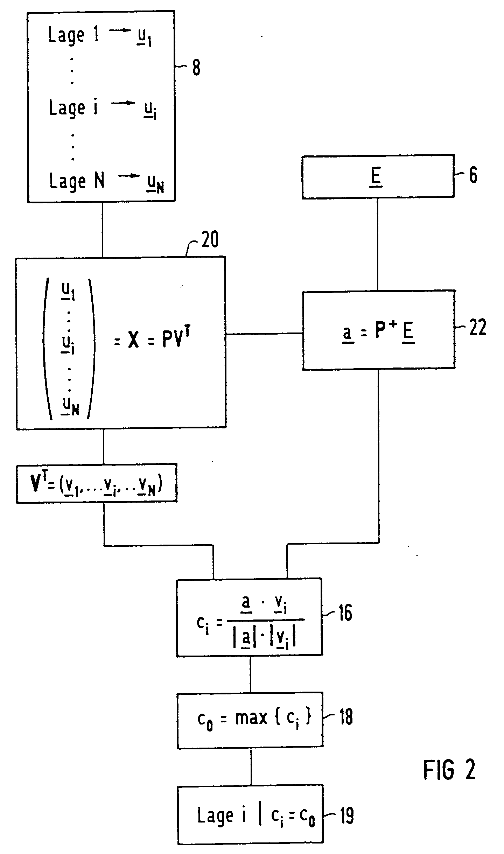

Eine Datenreduktion sowohl der Vergleichs-Werte $\underline{u}_i$ wie der Meßwerte $\underline{E}$ kann, wie in FIG 2 in Funktionsblöcken dargestellt ist, durch eine spektrale Zerlegung der Vektoren $\underline{u}_i$ und $\underline{E}$ nach dem Prinzip der Hauptkomponentenzerlegung (principal component analysis) erfolgen. Dabei wird von einer Matrix $\mathbf{X}$ ausgegangen, die die in der Datenbank 8 abgelegten Vergleichs-Oberflächenpotentiale $\underline{u}_i$ enthält. Für die Matrix $\mathbf{X}$ wird eine Sigulärwertzerlegung 20 durchgeführt, so daß die Matrix $\mathbf{X}$ sich darstellt als ein Matrizenprodukt

$$X = USV^T.$$

Dabei ist $\mathbf{U}$ eine orthogonale $L \times L$ Matrix, die die Eigenvektoren von $\mathbf{XX}^T$. $\mathbf{V}$ ist eine orthogonale $N \times N$ Matrix, die die Eigenvektoren von $\mathbf{X}^T\mathbf{X}$ enthält, wobei $\mathbf{V}^T = (\underline{V}_1,...\underline{V}_N)$ ist. $\mathbf{S}$ ist die $L \times N$ Matrix der Singulärwerte. Definiert man $P = US$, so gilt

$$X = PV^T$$

Die $L \times N$ Matrix $\mathbf{P} = (\underline{P}_1,...\underline{P}_N)$ enthält die N Eigenvektoren des Signalraums von $\mathbf{XX}^T$, die sogenannten principal components. Die spektrale Zerlegung eines Signalvektors $\underline{u}_i$ lautet demnach

$$\underline{u}_i = \sum_{l=1}^{N} V_{il} \times \underline{P}_l \; ; \; (1 \leq i \leq N).$$

Der Meßwertvektor $\underline{E}$ wird entsprechend einer spektralen Zerlegung unterworfen. Dazu muß die verallgemeinerte Inverse oder Moore Penrose Inverse $\mathbf{P}^+$ der Matrix $\mathbf{P}$ der N Eigenvektoren des Signalraums von $\mathbf{XX}^T$ gebildet werden. Die Meß-Entwicklungkoeffizenten $\underline{a}$

werden dann entsprechend über eine Matrizenmultiplikation 24 gebildet:

$$\underline{a} = P^+\underline{E}.$$

Der Vergleich über eine Korrelation 16 erfolgt entsprechend wie schon anhand von FIG 1 beschrieben, indem hier Skalarprodukte $c_i$ der Meß-Entwicklungskoeffizienten $\underline{a}/|\underline{a}|$ des Meßwertvektors $\underline{E}$ mit den normierten Vergleichs-Entwicklungskoeffizienten $\underline{V}$ /$|\underline{V}_i|$ gebildet werden. Danach wird der größte Korrelationskoeffizient $C_0$ herausgesucht. Die Lage der dazugehörigen Vergleichs-Herzaktivität wird dann als Lokalisierungsergebnis ausgegeben.

**Patentansprüche**

1. Verfahren zum Lokalisieren einer elektrischen Herzaktivität, bei dem

   - mit einem Vielkanalmeßsystem (4,6) von der Herzaktivität erzeugte Körperoberflächenpotentiale an mehreren Meßpunkten gemessen werden und Werte gespeichert werden, die die Körperoberflächenpotentiale an den Meßpunkten charakterisieren,
   - die Werte mit in einer Datenbank (8) abgespeicherten Verqleichs-Werten verglichen werden, wobei die Vergleichs-Werte Vergleichs-Oberflachenpotentiale charakterisieren, die von Vergleichs-Herzaktivitäten herrühren, deren Lage im Herzen bekannt ist, und bei dem
   - die Lage derjenigen Vergleichs-Herzaktivität als Lokalisierungsergebnis der Herzaktivität ausgegeben wird, deren Vergleichs-Werte mit den charakteristischen Werten die größte Ähnlichkeit aufweisen, **dadurch gekennzeichnet,** daß die Vergleichswerte mit Hilfe eines in einem Thoraxmodell (14) angeordneten Herzmodells (12) ermittelt worden sind.

2. Verfahren nach Anspruch 1, **dadurch gekennzeichnet,** daß zum Vergleich normierte Korrelationskoeffizienten (16) aus den charakterisierenden Werten mit den Vergleichswerten jeder Vergleichs-Herzaktivität gebildet werden, wobei die Lage der Vergleichs-Herzaktivität, deren Vergleichs-Werte mit den charakterisierenden Werten den größten Korrelationskoeffizienten bildet, als Lokalisierungsergebnis ausgegeben wird.

3. Verfahren nach Anspruch 1 oder 2, **dadurch gekennzeichnet,** daß der Vergleich auf den QRS-Komplex in den Körperoberflächenpotentialen beschränkt ist.

4. Verfahren nach einem der Ansprüche 1 bis 3, **dadurch gekennzeichnet,** daß die charakteristischen Werte den Zeitverlauf der Körperoberflächenpotentiale an den Meßpunkten wiedergeben.

5. Verfahren nach Anspruch 4, **dadurch gekennzeichnet,** daß aus diskreten Werten des Zeitverlaufs der Körperoberflächenpotentiale an den Meßpunkten Komponenten eines Meßwertvektors (6) gebildet werden, daß für jede Lage (10) der Vergleichs-Herzaktivität ein entsprechender Vergleichsvektor vorliegt, daß der Meßwertvektor mit jedem Vergleichsvektor verglichen wird, indem jeweils ein auf die Beträge der beiden Vektoren normiertes Skalarprodukt der beiden Vektoren gebildet wird, und daß die Lage der Vergleichs-Herzaktivität, deren Vergleichsvektor mit dem Meßwertvektor das größte normierte Skalarprodukt bildet, als Lokalisierungsergebnis ausgegeben wird.

6. Verfahren nach einem der Ansprüche 1 bis 3, **dadurch gekennzeichnet,** daß aus dem disketisierten zeitlichen Verlauf von Vergleichs-Oberflächenpotentialen, die von Vergleichs-Herzaktivitäten herrühren, deren Lagen im Herzen bekannt sind, Eigenvektoren extrahiert werden, daß die charakterisierenden Werte Meß-Entwicklungskoeffizienten wiedergeben, die das Ergebnis einer spektralen Zerlegung des diskretisierten zeitlichen Verlaufs der an den Meßpunkten gemessenen Körperoberflächenpotentiale nach den Eigenvektoren darstellen, und daß die Vergleichs-Werte Vergleichs-Entwicklungskoeffizienten wiedergeben, die das Ergebnis einer spektralen Zerlegung des diskretisierten zeitlichen Verlaufs der Vergleichs-Oberflächenpotentiale sind.

FIG 1

FIG 2